# EUROPEAN PATENT APPLICATION

(11) **EP 1 559 445 A1**
(43) Date of publication of application: **03.08.2005**
(21) Application number: 03715520.7
(22) Date of filing: 27.03.2003
(51) Int. Cl.: A61N 1/40, A61N 5/02

(54) **CYTOKINE CONTROLLING DEVICE, TREATING DEVICE AND TREATING METHOD**

(30) Priority: 27.03.2002 JP 2002088013
(71) Applicant: Nippon Sigmax Co., Ltd., Bunkyo-ku, Tokyo 112-0004 (JP)
(72) Inventor: AOKI, Takashi, Kasugai-shi, Aichi 487-0006 (JP); ARAHATA, Susumu, Suginami-ku, Tokyo 168-0063 (JP); SHINNABE, Hideyuki, Sagamihara-shi, Kanagawa 228-0802 (JP); KIKUKAWA, Tadahiro, Saitama-shi, Saitama 331-0061 (JP)
(74) Representative: Kirschner, Klaus Dieter
(86) International application number: PCT/JP2003/003843
(87) International publication number: WO 2003/080179

(57) **Abstract**

An electromagnetic wave emitting unit (1) formed by winding an emission coil (1-2) around a coil bobbin (1-1) is provided in a case (2). A current (for example, resonance frequency of 60 kHz, resonance current of 0.95 mA) is supplied from a power supply unit (3) to the emission coil (1-2) via a resonance circuit (4). With this operation, weak electromagnetic waves are emitted from the emission coil (1-2) to form an electromagnetic field (for example, magnetic flux density ≒ 6.7 nT, electric field strength ≒ 0.42 V/m) suitable for treating at a P-point. When an affected part is set at the P-point and in the vicinity thereof, a cytokine secretion is controlled. It is confirmed that inflammatory cytokines in an animal subjected to inflammation, for example, are significantly controlled. Efficacy is recognized in a range of 20 to 180 kHz, and especially a preferable result is obtained in the vicinity of 60 kHZ.

## Description

### Background of the Invention

The present invention relates to a cytokine controlling device which controls cytokines secreted in an organism and a treating device using its control function, for example, an inflammation treating device which inhibits inflammatory cytokines.

An organism secretes cytokines in response to an invasion (an attack or stimulus that may disturb the internal environment of the organism) to survive by maintaining its homeostasis. At this time, a vital reaction is caused by cytokines in the organism. For example, when inflammatory cytokines are secreted in response to a stimulus that causes an inflammation, an inflammatory reaction occurs in the organism.

As inflammatory cytokines, TNF (Tumor Necrosis Factor)-α, IL (Interleukin)-1β, IL-6, IL-8, and the like are known. Note that cytokines are described in detail in reference 1 ("Cytokines and Diseases", Bessatu Igaku No Ayumi, Jiro Imanishi (Editor), Katsuji Fujita (Publisher), Ishiyaku Publishers, In., (Publishing Office), First Edition/First Printing: Published July 10, 2000), and hence a detailed description thereof will be omitted. Conventionally, in most cases, as a means for promoting or inhibiting the secretion of cytokines, some kinds of chemical substances such as medicines and biological components are applied to receptors existing in nerves and cells associated with secretion.

There are many studies on the role of cytokines on inflammation. Cytokines such as TNF-α (Tumor Necrosis Factor-α) and IL-1 are produced by various kinds of cells including immunocompetent cells in response to cellular stresses such as infection (Koj, A., Biochim. Biophys. Acta, 1317, 84 - 94 (1996)). The biological activities of such cytokines include both a positive regulating mechanism and a negative regulating mechanism. In a normal state, the regulating mechanisms work in a balanced manner to constitute a network to maintain the homeostasis of the organism. It is thought that when a proper amount of cytokines exist, they play an important role in immune reactions, whereas excessive production of cytokines is associated with inflammatory diseases (Dinarello, C.A., Curr. Opin. Immunol., 3, 941 - 948 (1991)). If, for example, the negative regulating mechanism (control mechanism) for tumor necrosis factor (TNF-α) does not function sufficiently, TNF-α promotes the production of other various kinds of inflammatory cytokines such as IL-1β, IL-6, and IL-8, and the produced cytokines promote the production of TNF-α. This chain reaction creates a vicious circle of inflammation. This causes the destruction of tissues in bones, cartilages, and the like, resulting in articular rheumatism as autoimmune disease and the like.

Studies have been made on methods of inhibiting cytokines. Japanese Patent Laid-Open No. 2002-363104 reported an inflammatory cytokine inhibitor based on a nonsteroidal anti-inflammatory agent. Japanese Patent Laid-Open No. 2002-326950 reported an inflammatory cytokine inhibitor containing lactoferrin as an active ingredient. In addition, Japanese Patent Laid-Open No. 2001-114690, PCT (WO) 7-50317 (WO 95/14081), PCT (WO) 9-505055 (WO 95/13067) reported a p38 MAP kinase inhibitor. Japanese Patent Laid-Open No. 11-180873 reported an NF-κB activation inhibitor. Japanese Patent Laid-Open No. 2000-239182 reported an inflammatory cytokine control agent containing a hepatocyte growth factor as an active ingredient. All these reports are associated with methods using medicines and extracts, and hence there is a risk of excess inhibition, resistance occurrence, and the side effects of medicines. In addition, inflammatory diseases such as autoimmune diseases induced by inflammatory cytokines tend to become chronic, and hence the patients require long-term medical treatment. Symptomatic treatment using a nonsteroidal agent, from which no complete cure can be expected, is mainly applied to such diseases. Therefore, such a method is not suitable for the use of medicines having side effects.

A method of controlling cytokines without any medicines is reported, in which a proper amount of oxidative stress is imposed on a homologous T-cell suspension to cause a gas mixture of ozone and oxygen to foam, or cells are irradiated with UV light in addition to the imposition of the stress, thereby inducing a reduction in the production of inflammatory cytokines in T-cells and a reduction in hyperplastic reaction (PCT (WO) 2002-523332). A method is reported, in which a voltage within the range in which no electrolysis occurs or a voltage or magnetic field equal to or higher than a voltage at which electrolysis occurs is applied to blood during extracoporal circulation inside a filter, and the blood is caused to pass between electrodes or magnetic fields to forcibly release ion channels on the surfaces of cells such as leucocytes, changes in cell membrane potential, or pumps on cell surfaces by stimulating the cells owing to the effect of the voltage, current, or magnetic field, thereby activating the cells and producing or releasing Interleukin and interferon as cytokines (PCT (WO) 8-508299). Japanese Patent Laid-Open No. 11-322619 discloses a method in which human blood or lymph is caused to pass between electrodes and electric fields to forcibly release ion channels on the surfaces of cells such as leucocytes, changes in cell membrane potential, pumps on cell surfaces, or the like by stimulating the cells owing to the effects of a voltage and current, thereby activating the cells and producing Interleukin and interferon as cytokines.

The descriptions about all these cytokine control methods which do not depend on medicines concern methods of processing cells in vitro which are extracted in vitro. Therefore, they are not noninvasive treating methods for organisms.

### Summary of the Invention

It is an object of the present invention to provide a cytokine controlling device which controls the secretion of cytokines secreted from cells in organisms by using the effects of electromagnetic waves or electromagnetic fields without any mediacy of chemical substances such as medicines or biological components.

It is another object of the present invention to provide a treating device which can avoid the side effects of chemical substances such as medicines and phytotoxicity by controlling cytokines using weak electromagnetic wave or electromagnetic stimuli with respect to organisms because there is a risk of side effects or phytotoxicity when cytokines are to be inhibited by chemical substances such as medicines.

In order to achieve the above objects, the present invention includes electromagnetic wave emitting means for emitting electromagnetic waves upon reception of power, and power supply means for supplying power to the electromagnetic wave emitting means. This makes it possible to greatly inhibit inflammatory cytokines secreted in an organism by emitting weak electromagnetic waves of, for example, 20 to 180 kHz (preferably, 60 kHz) and applying an electromagnetic field formed by the electromagnetic waves to the organism.

Studies on cytokine controlling methods for organisms have been mainly those which use medicines, and there have been no report about noninvasive cytokine control using electric fields and magnetic fields. By irradiating an organism with electromagnetic waves from outside the organism using the power supply means and electromagnetic wave emitting means of the present invention, the amounts of cytokines in the organism can be noninvasively adjusted.

Note that the frequency of electromagnetic waves emitted from the electromagnetic wave emitting means is not limited to 20 to 180 kHz as long as electromagnetic waves can control cytokines. That is, in the present invention, the possible frequency range of electromagnetic waves emitted from the electromagnetic wave emitting means varies to some extent. The strengths of magnetic fields and electric fields can be adjusted by a resonance frequency (fr).

In addition, the present invention can control the amounts of cytokines secreted in an organism and select a cytokine to be controlled by changing the frequency or strength of electromagnetic waves, and hence can be used as a test device or a device for research (cytokine controlling device) as well as a treating device.

If, for example, the inflammatory cytokine inhibiting effect is used, diseases that can be treated include rheumatoid arthritis, multiple myeloma, Castleman's disease, Crohn's disease, myocarditis, myocardial infarct, arterial sclerosis, spondylitis, and spinal cord injury. However, the present invention is not limited to them, and can be applied to any diseases for which curative effects can be provided by cytokine control.

As the electromagnetic wave emitting means, a coil is generally used. However, an antenna or the like can be used. In addition, a resonance circuit may be provided to feed a resonance current into the electromagnetic wave emitting means by adjusting the frequency of electromagnetic waves to the resonance frequency of the resonance circuit. As electromagnetic waves to be emitted, continuous waves such as rectangular waves, complex waves, and band noise, or waves obtained by converting them into pulse-like waves may be used.

### Brief Description of Drawings

Fig. 1 is a view showing the basic arrangement of a cytokine controlling device according to an embodiment of the present invention;
Fig. 2 is a block diagram schematically showing the internal circuit arrangement of the power supply unit of this cytokine controlling device;
Fig. 3 is a graph for explaining the inhibiting effects of irradiation with electromagnetic waves from the cytokine controlling device with respect to inflammatory cytokines TNF-α and IL-6;
Fig. 4A is a view exemplifying a method of changing a resonance frequency by using a resonance coil with intermediate taps;
Fig. 4B is a view exemplifying a method of changing the resonance frequency by using a resonance capacitor group;
Fig. 4C is a view exemplifying a method of changing the resonance frequency by using a resonance coil group;
Fig. 4D is a view exemplifying a method of changing the resonance frequency by using a series circuit group constituted by resonance coils and resonance capacitors;
Fig. 5 is a perspective view showing an example of how emission coils are mounted when electromagnetic waves with difference frequencies are to be simultaneously emitted;
Fig. 6A is a plan view showing an example of how an annular coil (toroidal coil) is used as an emission coil;
Fig. 6B is a perspective view exemplifying a emitting antenna (rod antenna) which can be used in place of an emission coil;
Fig. 6C is a sectional view exemplifying an emitting antenna (flat antenna) which can be used in place of the emission coil;
Fig. 7 is a view showing a measurement result on IL-6 in the blood serum of collagen-induced arthritis rat models which is obtained while the frequency of electromagnetic waves from the cytokine controlling device is changed;
Fig. 8 is a view showing a measurement result on anti-type II collagen antibody titer in the blood serum of collagen-induced arthritis rats which is obtained while the frequency of electromagnetic waves from the cytokine controlling device is changed;
Fig. 9 is a view showing a test result on the arthritis tissues of MRL spontaneous arthritis model mice upon irradiation with electromagnetic waves from the cytokine controlling device;
Fig. 10 is a view showing a test result on the arthritis tissues of type II collagen-induced arthritis model mice upon irradiation with electromagnetic waves from the cytokine controlling device; and
Fig. 11 is a view showing a test result on the arthritis tissues of type II collagen-induced arthritis model mice upon irradiation with electromagnetic waves with different frequencies from the cytokine controlling device.

### Detailed Description of the Preferred Embodiment

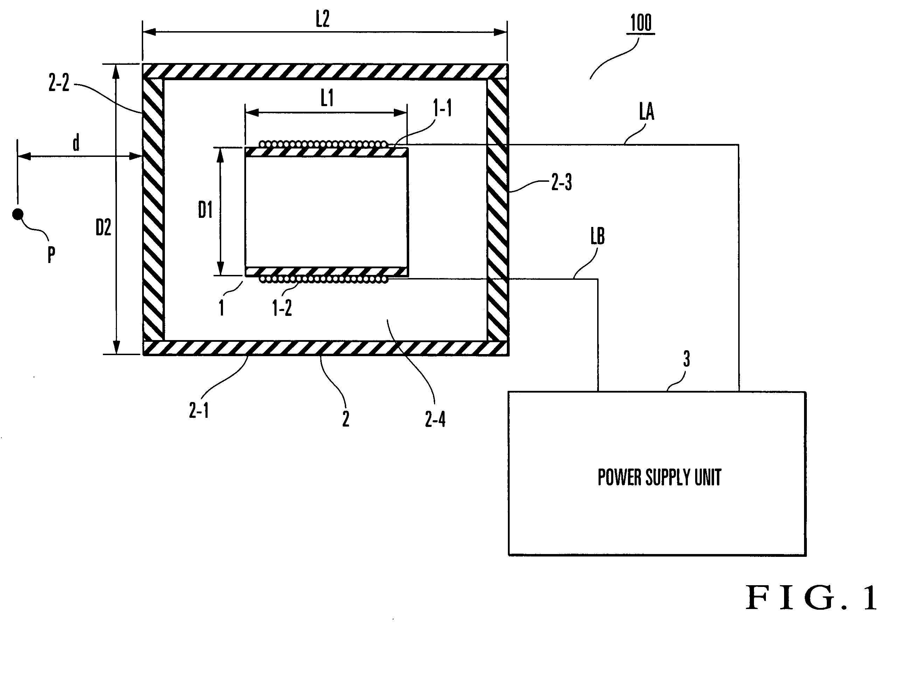

The present invention will be described below with reference to the accompanying drawings. Fig. 1 is a view showing the basic arrangement of a cytokine controlling device according to an embodiment of the present invention. Referring to Fig. 1, reference numeral 1 denotes an electromagnetic wave emitting unit; 2, a case housing the electromagnetic wave emitting unit 1; and 3, a power supply unit which supplies power to the electromagnetic wave emitting unit 1.

The electromagnetic wave emitting unit 1 is comprised of a cylindrical coil bobbin 1-1 and a coil (emission coil) 1-2 wound around the coil bobbin 1-1. The case 2 is comprised of a cylindrical case body 2-1 and disks 2-2 and 2-3 provided on the front and rear surfaces of the case body 2-1. The coil bobbin 1-1, case 2-1, and disks 2-2 and 2-3 are made of vinyl chloride. The electromagnetic wave emitting unit 1 is fixed with mounting members (not shown) so as to be located almost in the center of an internal space 2-4 of the case 2. The emission coil 1-2 of the electromagnetic wave emitting unit 1 is may be integrated with the power supply unit 3 or separated therefrom with extended leads 1A and LB.

A diameter D1 and length L1 of the coil bobbin 1-1 are respectively set to about 5 cm and about 7 cm. A diameter D2 and length L2 of the case body 2-1 are respectively set to about 10 cm and about 15 cm. The emission coil 1-2 is formed by one layer and its number of turns is set to 45. One end and the other end of the emission coil 1-2 are extended from the disk 2-3 side to the outside of the case 2 through the leads LA and LB.

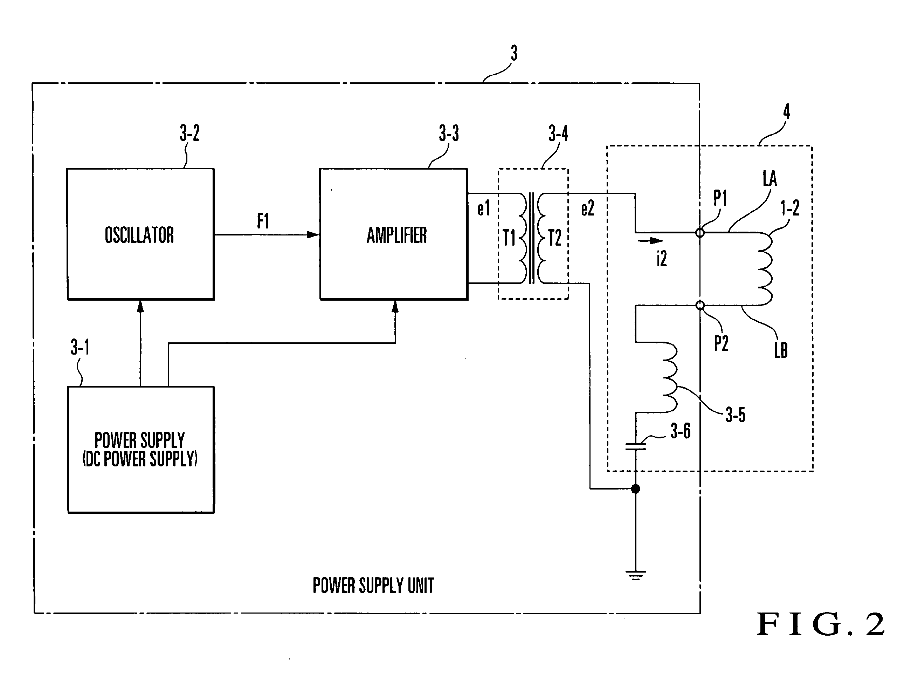

Fig. 2 schematically shows the internal circuit arrangement of the power supply unit 3. The power supply unit 3 includes a power supply (DC power supply) 3-1, an oscillator 3-2 which generates an oscillation signal F1 upon reception of power supplied from the power supply 3-1, an amplifier 3-3 which amplifies the oscillation signal F1 from the oscillator 3-2 upon reception of power supplied from the power supply 3-1, a step-up transformer 3-4 which is comprised of a primary winding T1 and secondary winding T2, a resonance coil 3-5, and a resonance capacitor 3-6.

The step-up transformer 3-4 receives the oscillation signal F1 amplified by the amplifier 3-3 as an input AC voltage e1 to the primary winding T1, raises the input AC voltage e1, and outputs the resultant voltage as an output AC voltage e2 from the secondary winding T2. Assume that in this embodiment, the output AC voltage e2 output from the secondary winding T2 has a sine waveform. Although not shown, the oscillator 3-2 has an adjustment knob. The frequency of the oscillation signal F1 can be adjusted by operating this adjustment knob.

The lead LA from one end of the emission coil 1-2 of the electromagnetic wave emitting unit 1 is connected to one end of the secondary winding T2 of the step-up transformer 3-4 through a terminal P1 in the power supply unit 3. The lead LB from the other end of the coil 1-2 is connected to one end of the resonance coil 3-5 through a terminal P2. The other end of the secondary winding T2 of the step-up transformer 3-4 is grounded. The other end of the resonance coil 3-5 is grounded through the resonance capacitor 3-6.

In this embodiment, the inductance of the resonance coil 3-5 is set to 150 mH, and the capacitance of the resonance capacitor 3-6 is set to 47 pF. The emission coil 1-2, resonance coil 3-5, and resonance capacitor 3-6 constitute a series resonance circuit 4 in which an AC current i2 flowing therein exhibits a maximum value when the frequency of the AC voltage e2 becomes 60 kHz. The series resonance circuit 4 is formed such that a resonance current flows when a resonance frequency fr is set to 60 kHz, and the frequency of the AC voltage (AC power) e2 becomes 60 kHz.

In this embodiment, the frequency of the oscillation signal F1 in the oscillator 3-2 is adjusted in advance such that the AC current i2 exhibits a maximum value while an increase in the AC current i2 flowing in the series resonance circuit 4 is monitored by an ammeter. By this adjustment, the value of the output AC voltage e2 output from the secondary winding T2 of the step-up transformer 3-4 is set to 5.6 V; the value of the AC current i2 flowing in the series resonance circuit 4, 0.95 mA; and the frequencies of the output AC voltage e2 and AC current i2, 60 kHz.

### [Emission of Electromagnetic Waves]

Referring to Fig. 2, the power supply (DC power supply) 3-1 is turned on to supply power from the power supply 3-1 to the oscillator 3-2 and amplifier 3-3. The oscillator 3-2 generates the oscillation signal F1 upon reception of power supplied from the power supply 3-1, and sends it to the amplifier 3-3. The amplifier 3-3 amplifies the oscillation signal F1 from the oscillator 3-2 and sends it to the step-up transformer 3-4.

The step-up transformer 3-4 receives the oscillation signal F1 amplified by the amplifier 3-3 as the input AC voltage e1 to the primary winding T1, raises the input AC voltage e1, and outputs it as the output AC voltage e2 from the secondary winding T2. With this operation, the AC current i2 flows in the emission coil 1-2 of the electromagnetic wave emitting unit 1, and the emission coil 1-2 emits electromagnetic waves.

At this time, since the frequency of the AC voltage e2 is set to 60 kHz in accordance with the frequency of the oscillation signal F1, which has been adjusted in advance, and coincides with the resonance frequency fr of the series resonance circuit 4, the AC current i2 flowing in the emission coil 1-2 becomes maximized. Therefore, the emission coil 1-2 emits electromagnetic waves at the maximum efficiency. That is, feeding an alternating current with the resonance frequency fr into the emission coil 1-2 raises the voltage applied to the emission coil 1-2, thus emitting an electric field together with a magnetic field at a high efficiency.

The resonance frequency fr is obtained by fr = 1/[2π(LC)^{1/2}) where L is the inductance of the resonance coil and C is the capacitance of the resonance capacitor. As the product of L and C remains constant, the resonance frequency fr remains unchanged. When the inductance L is increased and the capacitance C is decreased, Q representing the sharpness of the circuit increases, resulting in a large electric field. In contrast to this, when the inductance L is decreased and the capacitance C is increased, Q representing the sharpness of the circuit decreases, resulting in a small electric field. In this manner, the strength ratio between an electric field and a magnetic field can be changed. In addition, since Q of the circuit is inversely proportional to the DC resistance of the resonance circuit, the strengths of an electric field and magnetic field can be adjusted by inserting a variable resistor on the ground side of the resonance circuit.

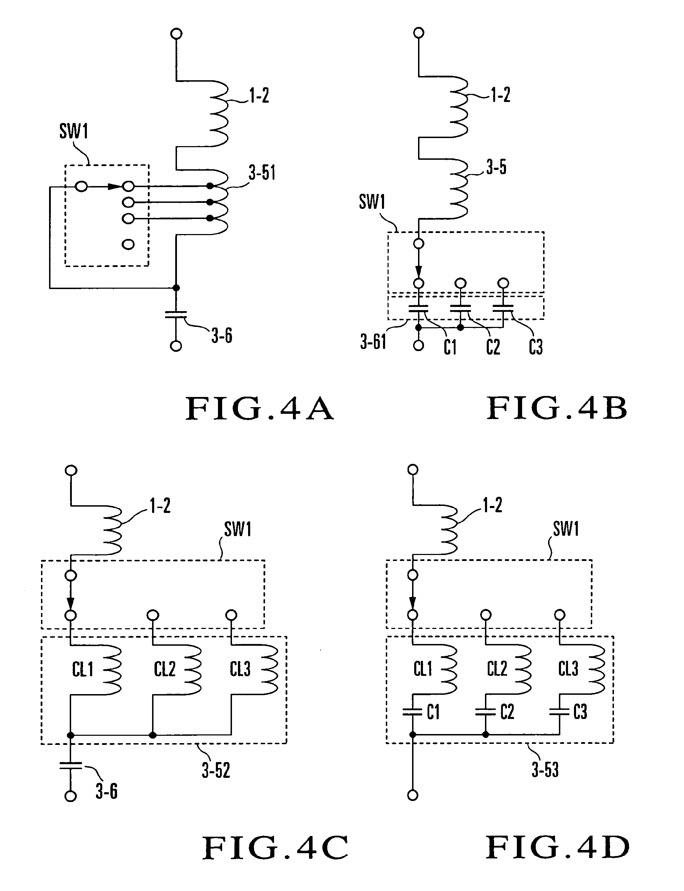

Methods of changing the resonance frequency fr include, for example, a method using a resonance coil 3-51 with intermediate taps as shown in Fig. 4A, a method using a resonance capacitor group 3-61 as shown in Fig. 4B, a method using a resonance coil group 3-52 as shown in Fig. 4C, and a method using a series circuit group 3-53 constituted by a resonance coil and resonance capacitor as shown in Fig. 4D.

Referring to Fig. 4A, the resonance frequency fr is changed by switching the positions of the intermediate taps of the resonance coil 3-51, which is connected to one end of the resonance capacitor 3-6, using a switch SW1.

Referring to Fig. 4B, the resonance frequency fr is changed by switching capacitors C1, C2 and C3 having different capacitances in the resonance capacitor group 3-61 connected to the resonance coil 3-5 using the switch SW1.

Referring to Fig. 4C, the resonance frequency fr is changed by switching coils CL1, CL2, and CL3 having different inductances in the resonance coil group 3-52 connected between the emission coil 1-2 and the resonance capacitor 3-6 using the switch SW1.

Referring to Fig. 4D, the resonance frequency fr is changed by series-connecting the coil CL1 and capacitor C1, the coil CL2 and capacitor C2, and the coil CL3 and capacitor C3, respectively, and switching the series-connected circuits constituted by the coils and capacitors using the switch SW1.

In the circuit arrangement shown in Fig. 2, the series resonance circuit 4 has the effect of attenuating the distortion component of the amplifier 3-3 and attenuating components other than a resonance frequency component. Obviously, if the signal from the amplifier 3-3 which is obtained by amplifying the oscillation signal F1 from the oscillator 3-2 contains few components other than a resonance frequency component, the resonance coil 3-5 and resonance capacitor 3-6 can be omitted.

In addition, in the circuit arrangement shown in Fig. 2, the ratio of a magnetic field to an electric field in the electromagnetic field formed at the P-point is set to about 1 : 0.044 by feeding a resonance current into the emission coil 1-2. The ratio of a magnetic field to an electric field at which a similar curative effect can be obtained varies to some extent, and a resonance current need not always be fed into the emission coil 1-2. That is, it suffices even if the frequency of the AC voltage e2 deviates from the resonance frequency fr or the AC voltage e2 of 60 kHz is applied to the emission coil 1-2 without using the resonance frequency. In addition, the strength of an electromagnetic field varies to some extent.

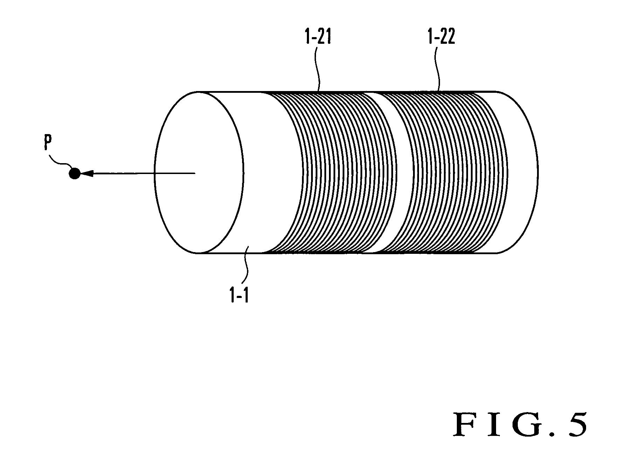

In this embodiment, the AC voltage e2 has a sine waveform, and electromagnetic waves having a sine waveform are emitted from the emission coil 1-2. However, continuous waves such as rectangular waves, complex waves, and band noise, or waves obtained by converting them into pulse-like waves may be used. For example, as shown in Fig. 5, a first emission coil 1-21 and second emission coil 1-2 may be wound around the coil bobbin 1-1 to simultaneously emit an electromagnetic wave having a frequency f1 and an electromagnetic wave having a frequency f2 (f1 ≠ f2) from the first emission coil 1-21 and second emission coil 1-22, respectively.

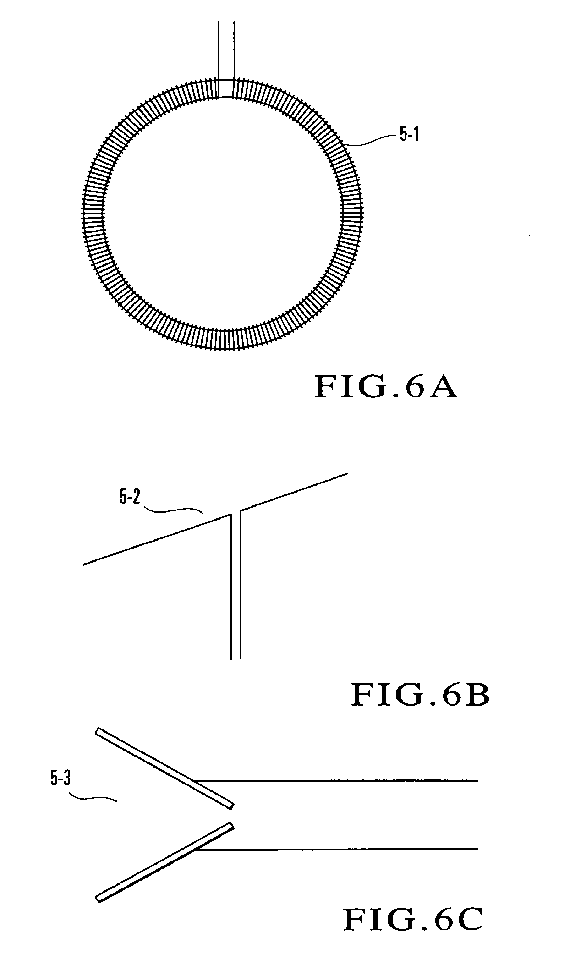

In this embodiment, the cylindrical emission coil 1-2 is used as a means for emitting electromagnetic waves. However, an annular coil (toroidal coil) 5-1 like the one shown in Fig. 6A, a rod-like antenna 5-2 like the one shown in Fig. 6B, or a flat antenna 5-3 like the one shown in Fig. 6C may be used.

The weak electromagnetic waves of 60 kHz emitted from the emission coil 1-2 are transmitted through the disk 2-2 to form an electromagnetic field in front of the disk 2-2. In this embodiment, an electromagnetic field with magnetic flux density ≒ 6.7 nT and electric field strength ≒ 0.42 V/m is generated at the P-point spaced apart from the disk 2-2 by a distance d (d = 50 cm). In the electromagnetic field at the P-point, the power density based on a magnetic field is about 0.011 (W/m²), and the power density based on an electric field is about 0.00047 (W/m²). The ratio of the magnetic field to the electric field is about 1 : 0.044. Electromagnetic waves with this ratio can be obtained by feeding a resonance current into the emission coil 1-2.

In this embodiment, the emission coil 1-2 has one layer. However, the coil is not limited to one layer, and may have two layers or more. In addition, a coil around which a pair of windings, e.g., parallel windings or twisted windings, are wound may be used. In this case, the magnitude of a magnetic field can be adjusted by shifting the phases of currents fed into one winding and the other winding from each other.

### [Animal Test ①: Test on Influence of Adjuvant-induced Arthritis of Rat on in vivo Cytokines]

### [Test Animal]

Five-week-old male SD rats were purchased (from Charles River Japan, Inc.) and used for experiments.

### [Breeding]

Each animal was housed and raised in a stainless steel bracket breeding cage. The temperature in each animal chamber was set to 22 ± 3°C (actual measurement value: 20 to 23°C); and the humidity, to 55 ± 15% (actual measurement value: 57 to 65%). An all fresh system was provided with a ventilation frequency of 10 times or more per hour. Indoor illumination was provided for 12 hrs from 6 am to 6 pm, with an illuminance of 150 to 300 Lux. As a feed, chow CE-2 (available from CLEA Japan, Inc.) was used, and the animals were allowed to liberally intake water.

### [Test Method]

### (1) Grouping

The animals were formed into two groups as indicated by ① and ② as follows, each consisting of five animals:
①: non-electromagnetic-wave-irradiated group (control group) ....group A
②: 60-kHz-electromagnetic-wave-irradiated group ....group B

An adjuvant solution (arthritis inducing substance) was administered to the above animal groups to examine the effect of repeated irradiation with 60-kHz electromagnetic waves from a cytokine controlling device 100. The period of irradiation for group B was 11 days.

### (2) Irradiation with 60-kHz Electromagnetic Waves

The animal was placed in polycarbonate mouse cage (215 W x 230 D x 130 H (unit: mm)) with chips being laid thinly. The animal was irradiated from directly above with 60-kHz electromagnetic waves from the cytokine controlling device 100. In this case, the animal was spaced apart from the device by 50 cm, i.e., was located at the P-point in Fig. 1. At this position, 10-min irradiation was repeatedly performed three times (a total of 30 min) in the morning with 20-min intermissions per day. Irradiation with 60-kHz electromagnetic waves was performed for a total of 11 days, i.e., 10 days before the administration of adjuvant and one day after the administration.

### (3) Production of Adjuvant-induced Arthritis

As an arthritis inducing substance, an adjuvant solution was prepared by suspending mycobacterium butyricum (Difco) in liquid paraffin (Kanto Kagaku). An adjuvant solution of 0.3 mg/0.05 ml/site was percutaneously injected into the footpad of the right hind leg to produce an arthritis rat.

### (4) Collection of Blood

Blood was collected by beheading each rat on the day following the administration of adjuvant. In accordance with a predetermined method, the blood was EDTA-treated and centrifuged to obtain blood plasma. The blood plasma was immediately frozen and used for cytokine measurement.

### (5) Measurement of Cytokines

The inflammatory cytokines TNF-α and IL-6 were measured by using commercially available kits (TNF-α: rat TNF-α, ELISA Kit Wako, Wako Pure Chemical Industries, Ltd, IL-6 (Endogen Rat Interleukin-6 ELISA, Endogen).

### [Test Result]

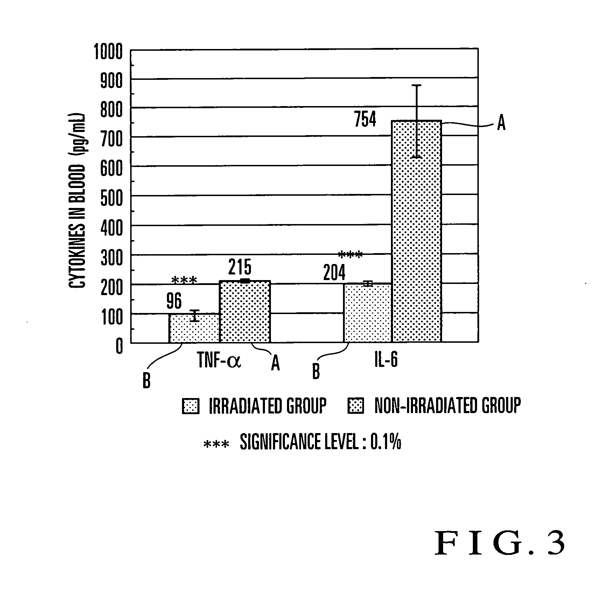

Fig. 3 shows the inhibition results of inflammatory cytokine TNF-α and IL-6 with irradiation with 60-kHz electromagnetic waves from the cytokine controlling device 100.

When the amounts of inflammatory cytokine TNF-α and IL-6 in groups A and B are compared with each other, the amount of TNF-α in non-electromagnetic-wave-irradiated group A is 215 pg/ml, and that in 60-kHz-electromagnetic-wave-irradiated group B is 96 pg/ml. It is therefore obvious that TNF-α in 60-kHz-electromagnetic-wave-irradiated group B is significantly inhibited as compared with non-electromagnetic-wave-irradiated group A (significance level: 0.1%). It is known that the inhibition of TNF-α is effective for the treatment of inflammatory diseases and rheumatoid arthritis.

The amount of IL-6 in non-electromagnetic-wave-irradiated group A is 754 pg/ml, and that in 60-kHz-electromagnetic-wave-irradiated group B is 204 pg/ml. Obviously, IL-6 in 60-kHz-electromagnetic-wave-irradiated group B is significantly inhibited as compared with non-electromagnetic-wave-irradiated group A (significance level: 0.1%). Since IL-6 is secreted by an amount corresponding to the degree of inflammation, the inhibition of IL-6 indicates that inflammation is inhibited.

It is known that this rat arthritis model has a strong correlation with human rheumatoid arthritis. It is strongly indicated that inhibiting TNF-α and IL-6 as inflammatory cytokines in this model is effective for the treatment of inflammatory diseases, rheumatoid arthritis, and the like in the human body.

### [Animal Test ②: Effects of Spontaneous Arthritis on Rheumatoid Factor and Tissues of MRL Mouse]

### [Test Animal]

Six-week-old MRL mice (MRL/MpJUmmCrj mice) subjected to spontaneous arthritis were purchased (from Charles River Japan, Inc.) and used for experiments. Note that MRL mice are described in reference 2 ("Arthritis Model", Chiyuki Abe and Takashi Sawai (Edition), Ishiyaku Publishers, In., (Publishing Office), First Edition/First Printing: Published July 19, 2000). MRL mice spontaneously develop arthritis without administration of any arthritis inducing substance.

### [Breeding]

The mice were raised in the same manner as in animal test ①.

### [Test Method]

### (1) Grouping

The animals were formed into two groups as indicated by ① and ② as follows, each consisting of six animals:
①: non-electromagnetic-wave-irradiated group (control group) six mice.....group C
②: 60-kHz-electromagnetic-wave-irradiated group six mice....group B

### (2) Irradiation with Electromagnetic Waves

The electromagnetic wave irradiation method was the same as in animal test ①. The period of irradiation with electromagnetic waves was five weeks from the start of the test; 10-min irradiation was repeatedly performed three times (a total of 30 min) in with 20-min intermissions per day.

### (3) Serologic Test/Histological Test

Blood was collected from the caudal vein the day before the start of a test and at the fifth test week to measure rheumatoid factors and anti-ds-DNA antibodies in the blood serum. After the experiment, according to a predetermined method, the right foreleg of each mouse was HE-stained, and a histological test was conducted.

### [Test Result]

### (1) Serologic Test Result

① Rheumatoid Factor RF-IgG
   The average amount of rheumatoid factor RF-IgG in non-electromagnetic-wave-irradiated group C was 120 Unit/ml, and that in
   60-kHz-electromagnetic-wave-irradiated group D was 73 Unit/ml. That is, RF-IgG was inhibited by irradiation with 60-kHz electromagnetic waves. Since RF-IgG is secreted in accordance with the degree of inflammation, the inhibition of RF-IgG indicates that inflammation is inhibited.
② Rheumatoid Factor RF-IgM
   The amount of rheumatoid factor RF-IgM in non-electromagnetic-wave-irradiated group C was 24 Unit/ml, and that in
   60-kHz-electromagnetic-wave-irradiated group D was 15 Unit/ml. That is, RF-IgM was inhibited by irradiation with 60-kHz electromagnetic waves. Since RF-IgM reflects the degree of acute-phase inflammation, this indicates that irradiation with 60-kHz electromagnetic waves inhibited acute inflammation.
③ Anti-ds-DNA Antibody
   The amount of anti-ds-DNA antibody that contributes to the transition from acute inflammation to chronic inflammation in
   non-electromagnetic-wave-irradiated group C was 1,457 Unit/ml, and that in
   60-kHz-electromagnetic-wave-irradiated group D was 737 Unit/ml. That is, anti-ds-DNA antibody was inhibited by irradiation with 60-kHz electromagnetic waves.

### (2) Histological Test Result

After the test, the animals were slaughtered. The right forelegs were then HE-stained and histological tissue examination was performed in accordance with a predetermined method. The samples were scored according to no change (-), slight change (+), moderate change (++), and serious change (+++) based on observation of superposition of synovial tissues, edematous change in subsynovial tissue, fibrin deposition, fibroblast proliferation, villus formation, cartilage destruction, cartilage hyperplasia, bone/cartilage connective tissue replacement, pannus formation, and bone neoplasm. In statistical analysis, a significant difference test was performed by using the Wilcoxon U test and chisquared test, and the significance level was set to p < 0.05.

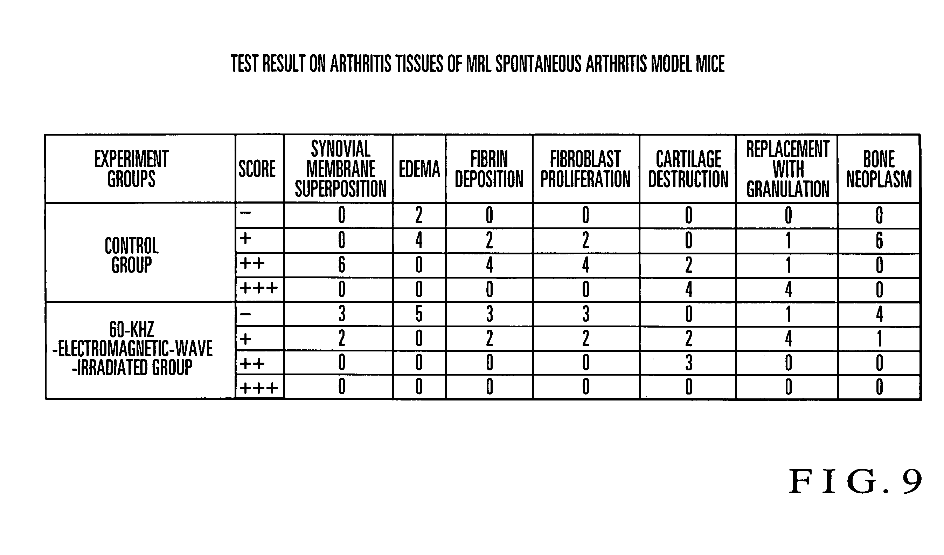

Fig. 9 shows the histological tissue examination result. In the non-electromagnetic-wave-irradiated group (control group), synovial membrane superposition, edema, fibrin deposition, fibroblast proliferation, cartilage destruction, replacement with granulation, and bone neoplasm were recognized, and hence the progression of the arthropathy was observed. In contrast to this, in the 60-kHz-electromagnetic-wave-irradiated group, the moderate synovial membrane superposition observed in the control group has been significantly improved. Likewise, edema, fibrin deposition, fibroblast proliferation, cartilage destruction, and replacement with granulation have been significantly improved. This MRL model is known to have a strong correlation with human rheumatoid arthritis. It is therefore believed that the cytokine controlling device is effective for the treatment of inflammatory diseases such as rheumatoid arthritis.

### [Animal Test ③: Effects on Arthritis Tissue Finding of Type II Collagen-Induced Arthritis Model Mouse]

### [Test Animal]

Six-week-old male DBA mice were purchased and used for experiments. Arthritis was produced by intracutaneously administering bovine type II collagen (Elastin Products Co., Inc.) into the tail head portions of the mice at 100 µg/head two times with one week's interval. Dexamethasone (ICN Biomedicals Inc.) was administered once a week, and the period of irradiation with electromagnetic waves was four weeks.

### [Test Method]

### (1) Grouping

The mice were formed into three groups each consisting of six mice as follows.

### (2) Irradiation with Electromagnetic Waves

①: non-electromagnetic-wave-irradiated group (control group)
②: dexamethasone-administered group(intramuscular administration of dexamethasone at 1 mg/kg once a week)
③: electromagnetic-wave-irradiated group (60-kHz-electromagnetic-wave-irradiated group): a total of 30-min irradiation every day, i.e., 10-min irradiation, 20-min intermission, 10-min irradiation, 20-min intermission, and 10-min irradiation

### (3) Histological Tissue Examination

After the test, the animals were slaughtered. The right forelegs were then HE-stained and histological tissue examination was performed in accordance with a predetermined method. The samples were scored according to no change (-), slight change (+), moderate change (++), and serious change (+++) based on observation of superposition of synovial tissues, edematous change in subsynovial tissue, fibrin deposition, fibroblast proliferation, villus formation, cartilage destruction, cartilage hyperplasia, bone/cartilage connective tissue replacement, pannus formation, and bone neoplasm. In statistical analysis, a significant difference test was performed by using the Wilcoxon U test and chisquared test, and the significance level was set to p < 0.05.

### [Test Result]

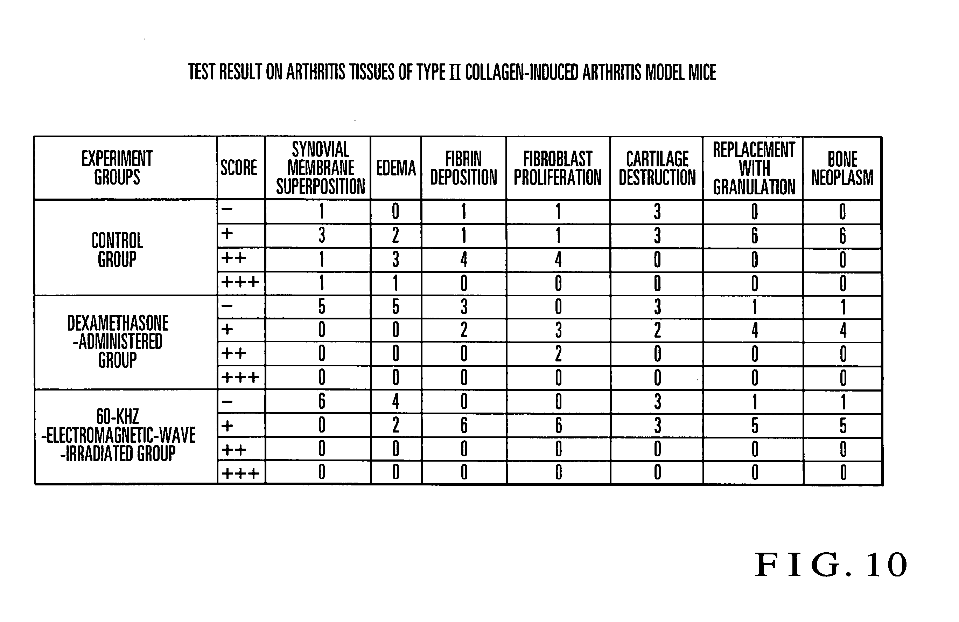

Fig. 10 shows the histological tissue examination result. In control group ①, deteriorations in the scores of synovial membrane superposition, edema, fibrin deposition, fibroblast proliferation, cartilage destruction, replacement with granulation, and bone neoplasm were recognized, and hence the progression of the arthropathy was observed. In dexamethasone-administered group ② and 60-kHz-electromagnetic-wave-irradiated group ③, significant improvements in the scores of synovial membrane superposition, edema, and replacement with granulation, of these changes, were recognized. With regard to fibrin deposition, fibroblast proliferation, cartilage destruction, and bone neoplasm, no significant changes were recognized in both dexamethasone-administered group ② and 60-kHz-electromagnetic-wave-irradiated group ③ as compared with non-electromagnetic-wave-irradiated group ①. No significant difference was observed between dexamethasone-administered group ② and 60-kHz-electromagnetic-wave-irradiated group ③. These results indicate that irradiation of the type II collagen-induced arthritis model mouse with electromagnetic waves has a curative effect similar to that of dexamethasone as an antirheumatic drug.

### [Animal Test Using Electromagnetic Waves with Different Frequencies]

### [Animal Test ④: Measurement on IL-6 in Blood Serum of Collagen-Induced Arthritis Rat Model and Anti-Type II Collagen Antibody Titer]

### [Test Animal]

Six-week-old female Lew rats were used.

### [Test Method]

### (1) Grouping

The rats were formed into seven groups each consisting of five rats: ① non-treated group (control group), ② disease control group, ③ dexamethasone-administered group, ④ electromagnetic-wave-irradiated group (20-kHz-electromagnetic-wave-irradiated group), ⑤ electromagnetic-wave-irradiated group (60-kHz-electromagnetic-wave-irradiated group), ⑥ electromagnetic-wave-irradiated group (180-kHz-electromagnetic-wave-irradiated group), and ⑦ electromagnetic-wave-irradiated group (540-kHz-electromagnetic-wave-irradiated group).

### (2) Production of Arthritis

An emulsion prepared by agitating 10 mg/ml of bovine type II collagen (Elastin Products Co., Inc.) in a 0.02 M tris/0.15 M salt buffer (pH: 8.0) solution was intracutaneously administered into the tail head portions of the rats in groups ② to ⑦ at 1 mg/head.

### (3) Electromagnetic Wave Irradiation Method

From the day before the administration of type II collagen, 10-min irradiation, 20-min intermission, 10-min irradiation, 20-min intermission, and 10-min irradiation were performed for the rats in groups ④ to ⑦ every day while the irradiation frequency of electromagnetic waves was adjusted to 20, 60, 180, and 540 kHz.

### (4) Drug Administration

Dexamethasone (ICN Biomedicals Inc.) having an anti-inflammatory effect was intramuscularly administered into the rats in dexamethasone-administered group ③ at 1 mg/head once a week from the day after the administration of type II collagen.

### (5) Measurement of IL-6 in Blood Serum

The blood serum of each rat was collected, and the amount of IL-6 in the blood serum was measured by using the rat IL-6 immunoassay kit available from ANALYZA.

### (6) Measurement on Anti-Type II Collagen Antibody Titer in Blood Serum

Rat blood serum was collected, and was measured by using a rat IgG anti-type II collagen ELISA kit (available from Chodrex).

### (7) Histological Tissue Examination

Histological tissue examination was executed in the same manner as in animal test ③.

### [Test result]

### (1) Measurement Result on IL-6

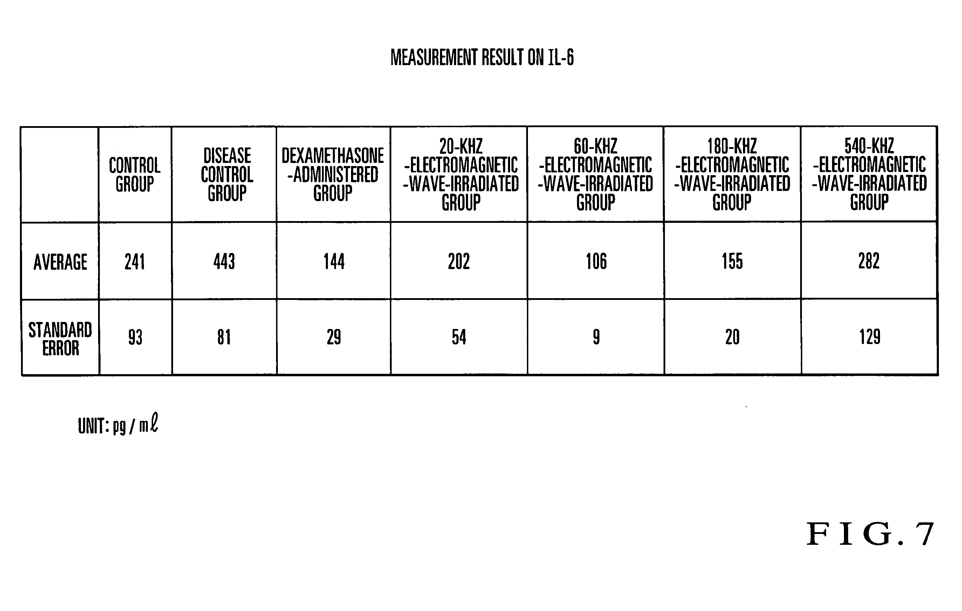

Referring to Fig. 7, in a chronic stage on the 42nd day of administration of type II collagen, the amount of IL-6 in non-treated group (control group) ① free from arthritis was 241 pg/ml. In contrast to this, in disease control group ②, for which no treatment was given in spite of the administration of type II collagen that is an arthritis inducing substance, the amount of IL-6 increased to 443 pg/ml.

In dexamethasone-administered group ③ into which dexamethasone, a kind of steroid known to have an anti-inflammatory effect, was intramuscularly administered at 1 mg/head per week, an increase in IL-6 was significantly suppressed to 144 pg/ml as compared with disease control group ②.

In 20-kHz-electromagnetic-wave-irradiated group ④, 60-kHz-electromagnetic-wave-irradiated group ⑤, 180-kHz-electromagnetic-wave-irradiated group ⑥, and 540-kHz-electromagnetic-wave-irradiated group ⑦, the measurement values of IL-6 in blood serums were 202, 106, 156, and 282 pg/ml, which were smaller than 443 pg/ml in disease control group ②. That is, it was confirmed that irradiation with electromagnetic waves had the effect of inhibiting an increase in inflammatory cytokine IL-6 in type II collagen inducing arthritis. Irradiation with 60-kHz and 180-kHz electromagnetic waves, in particular, exhibited a significant inhibitory effect equivalent to that in dexamethasone-administered group ③. In addition, irradiation with 20-kHz electromagnetic waves exhibited fairly significant inhibitory effect. In the present invention, the numerical limitations at 20 kHz to 180 kHz are based on this measurement result.

### (2) Measurement Result on Anti-Type II Collagen Antibody Titer

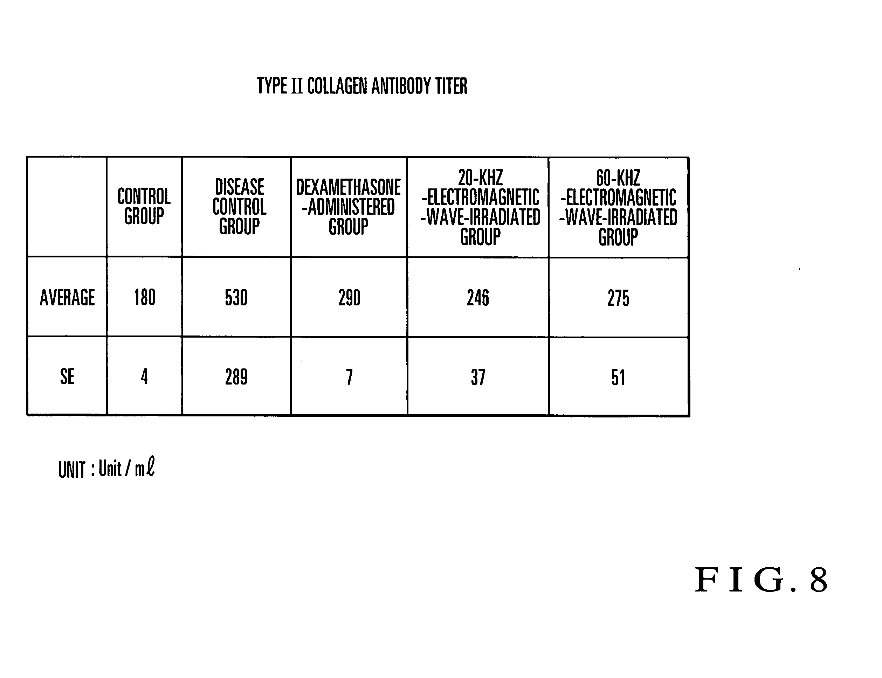

Referring to Fig. 8, the antibody titer in blood serum in control group ① into which no type II collagen was administered was 180 Unit/ml, whereas that in disease control group ② was 530 Unit/ml on the 42nd day of administration of type II collagen. In dexamethasone-administered group ③, an increase in antibody titer was significantly inhibited, and the amount of antibody titer was 290 Unit/ml. 20-kHz-electromagnetic-wave-irradiated group ④ and 60-kHz-electromagnetic-wave-irradiated group ⑤ exhibited the effect of inhibiting anti-type II collagen antibody titer almost equivalent to that exhibited by dexamethasone-administered group ③, and the amounts of antibody titers were 246 Unit/ml and 1,275 Unit/ml in the two groups, respectively.

### (3) Histological Tissue Examination Result

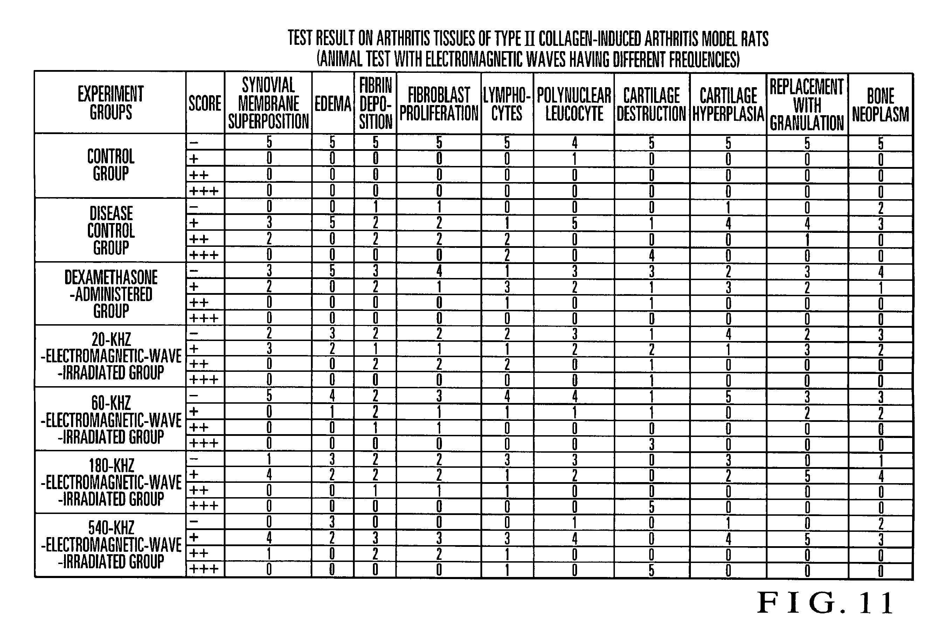

Fig. 11 shows a histological tissue examination result. As compared with non-treated control group ①, in disease control group ②, apparent tissue changes were recognized in test items such as superposition of synovial tissues, edema, fibrin deposition, fibroblast proliferation, lymphocytes, polynuclear leucocyte, cartilage destruction, cartilage hyperplasia, granulation replacement, and bone neoplasm. As compared with disease control group ②, in dexamethasone-administered group ③ and 20 to 180-kHz-electromagnetic-wave-irradiated groups ④, ⑤, and ⑥, apparent inflammatory tissue image improvements were recognized in many items. In 540-kHz-electromagnetic-wave-irradiated group ⑦, although an improvement tendency was recognized, a significant improved image was recognized only in item of edema. In 60-kHz-electromagnetic-wave-irradiated group ⑤, significant improvements were recognized in synovial membrane superposition, edema, lymphocytes, polynuclear leucocyte, cartilage destruction, cartilage hyperplasia, replacement with granulation, and the like, thus exhibiting effects equivalent to dexamethasone. When the respective groups are compared in terms of the number of items in which significant improvements were recognized, it was found that irradiation with 60-kHz electromagnetic waves and dexamethasone exhibited significant improvements in six items, i.e., the highest effect, and irradiation with 180-kHz electromagnetic waves, 20-kHz electromagnetic wave, and 540-kHz electromagnetic waves exhibited significant improvements in three items, two items, and one item, respectively.

In the medical treatment field, when a solid body or affected part with an inflammation such as an inflammatory disease or articular rheumatism is placed at or near the P-point in Fig. 1 and irradiated with electromagnetic waves from the cytokine controlling device (treating device) 100, inflammatory cytokines to be secreted are inhibited, and the inflammation is reduced. In this case, treatment for an inflammatory disease can be done without using any medicine or while reducing the amount of medicine with no risk of side effects and phytotoxicity. Although this treating device may be brought into contact with the body, the device can irradiate it at a proper distance from the device. The device is therefore characterized by being advantageous in terms of invasion, safety, and convenience.

As described above, the device according to the present invention considerably inhibits cytokines secreted in organisms and exhibits a strong inflammation inhibiting effect. In addition, it was confirmed from a plurality of sick animal models that this effect was equivalent to steroids as strong inflammation inhibitors widely used in the medical treatment field. Therefore, this device is expected to be effective for human inflammatory diseases, i.e., diseases caused by inflammatory cytokine TNF-α, IL-1β, IL-6, IL-8, and the like, e.g., rheumatoid arthritis, multiple myeloma, Castleman's disease, Crohn's disease, myocarditis, myocardial infarct, arterial sclerosis, spondylitis, and spinal cord injury.

In addition, this device can select a cytokine to be controlled by changing, for example, the frequency or strength of electromagnetic waves emitted from the emission coil, and can be used as a test device or a device for research as well as a treating device.

## Claims

1. A cytokine controlling device **characterized by** comprising electromagnetic wave emitting means for emitting electromagnetic waves upon reception of power, and power supply means for supplying power to said electromagnetic wave emitting means.

2. A cytokine controlling device according to claim 1, **characterized in that** said electromagnetic wave emitting means comprises a coil.

3. A cytokine controlling device according to claim 1, **characterized in that** said power supply means for said electromagnetic wave emitting means comprises an AC power supply.

4. A cytokine controlling device according to claim 3, **characterized by** further comprising a resonance circuit.

5. A cytokine controlling device according to claim 3, **characterized in that** a frequency of the electromagnetic waves is set to 20 to 180 kHz.

6. A cytokine controlling device according to claim 1, **characterized in that** the electromagnetic waves are noninvasive to an organism.

7. A treating device **characterized by** comprising electromagnetic wave emitting means for emitting electromagnetic waves upon reception of power, and power supply means for supplying power to said electromagnetic wave emitting means, wherein a cytokine is controlled by electromagnetic waves emitted from said electromagnetic wave emitting means.

8. A treating device according to claim 7, **characterized in that** an inflammatory cytokine is controlled by the electromagnetic waves.

9. A treating device according to claim 8, **characterized in that** a frequency of the electromagnetic waves is set to 20 to 180 kHz.

10. A treating device according to claim 7, **characterized in that** the electromagnetic waves are noninvasive to an organism.

11. A treating method which medically treats a disease by controlling a cytokine using electromagnetic waves.

12. A treating method according to claim 11, **characterized in that** the cytokine includes an inflammatory cytokine.

13. A treating method according to claim 12, **characterized in that** a frequency of the electromagnetic waves is set to 20 to 180 kHz.

14. A treating method according to claim 11, **characterized in that** the electromagnetic waves are noninvasive to an organism.

## Amended claims

### Amended claims under Art. 19.1 PCT

**1.** (amended) A cytokine controlling device **characterized by** comprising electromagnetic wave emitting means for emitting electromagnetic waves to an organism upon reception of power to control a cytokine in the organism, and power supply means for supplying power to said electromagnetic wave emitting means.

**2.** A cytokine controlling device according to claim 1, **characterized in that** said electromagnetic wave emitting means comprises a coil.

**3.** A cytokine controlling device according to claim 1, **characterized in that** said power supply means for said electromagnetic wave emitting means comprises an AC power supply.

**4.** A cytokine controlling device according to claim 3, **characterized by** further comprising a resonance circuit.

**5.** A cytokine controlling device according to claim 3, **characterized in that** a frequency of the electromagnetic waves is set to 20 to 180 kHz.

**6.** A cytokine controlling device according to claim 1, **characterized in that** the electromagnetic waves are noninvasive to an organism.

**7.** (amended) A treating device **characterized by** comprising electromagnetic wave emitting means for emitting electromagnetic waves to an organism upon reception of power to control a cytokine in the organism, and power supply means for supplying power to said electromagnetic wave emitting means.

**8.** A treating device according to claim 7, **characterized in that** an inflammatory cytokine is controlled by the electromagnetic waves.

**9.** A treating device according to claim 8, **characterized in that** a frequency of the electromagnetic waves is set to 20 to 180 kHz.

**10.** A treating device according to claim 7, **characterized in that** the electromagnetic waves are noninvasive to an organism.

**11.** A treating method which medically treats a disease by controlling a cytokine using electromagnetic waves.

**12.** A treating method according to claim 11, **characterized in that** the cytokine includes an inflammatory cytokine.

**13.** A treating method according to claim 12,
**characterized in that** a frequency of the electromagnetic waves is set to 20 to 180 kHz.

**14.** A treating method according to claim 11, **characterized in that** the electromagnetic waves are noninvasive to an organism.

Statement under Art. 19.1 PCT
Each of claims 1 and 7 comprises "electromagnetic wave emitting means for emitting electromagnetic waves to an organism upon reception of power to control a cytokine in the organism".

The present invention is based on a new finding that cytokines secreted in an organism can be controlled by emitting electromagnetic waves to the organism. Since prior art describing the control of cytokines by emitting the electromagnetic waves to the organism have not been disclosed, "electromagnetic wave emitting means for emitting electromagnetic waves to an organism upon reception of power to control a cytokine in the organism" is considered to be a new special technical feature. Therefore, at least claims 1 and 7, and claims according to these independent claims comprise a common special technical feature to satisfy the unity of invention.
